# EUROPEAN PATENT APPLICATION

(11) **EP 2 138 099 A2**
(43) Date of publication of application: **30.12.2009**
(21) Application number: 09008293.4
(22) Date of filing: 24.06.2009
(51) Int. Cl.: A61B 8/00, G06F 3/03

(54) **Ultrasound observation device and method for controlling operation thereof**

(30) Priority: 25.06.2008 JP 2008165815; 25.06.2008 JP 2008165930
(71) Applicant: FUJIFILM Corporation, Minato-ku Tokyo (JP)
(72) Inventor: Naruse, Mutsumi, Saitama-shi Saitama (JP)
(74) Representative: Klunker . Schmitt-Nilson . Hirsch

(57) **Abstract**

An ultrasound observation device (11) includes a depth knob (35, 40) and a pulse generator (35a, 40a). The depth knob (35, 40) is operated to switch the display depth of an ultrasound image on a monitor (21). Upon every rotation of the depth knob (35, 40) by a predetermined angle, the pulse generator (35a, 40a) generates a switching pulse. A control circuit (22) has a waiting time determiner (28), which determines a waiting time to start signal processing every time the switching pulse is generated. When a subsequent switching pulse is generated within the waiting time, the waiting time determiner (28) calculates an average generation interval of the switching pulses, and determines a new waiting time based on the average generation interval. When the waiting time elapsed without the generation of a subsequent switching pulse, the control circuit (22) recognizes the end of switching operation, and starts a display switching process under the conditions corresponding to the number of the switching pulses generated.

## Description

### FIELD OF THE INVENTION

The present invention relates to an ultrasound observation device to display an ultrasound image, and a method for controlling operation of the ultrasound observation device.

### BACKGROUND OF THE INVENTION

In the field of medicine, ultrasound diagnostic apparatus is used for diagnosis of those regions unsuitable for X-ray examination. A typical ultrasound diagnostic apparatus includes an ultrasound probe for transmitting ultrasound waves to a region in a patient's body cavity and receiving reflected ultrasound waves, or echoes, from that region, and an ultrasound observation device connected to the ultrasound probe. The ultrasound observation device applies various types of signal processing to the echo signal received by the ultrasound probe, and generates image data. Based on this image data, an ultrasound image is displayed on a monitor.

Generally, the ultrasound observation devices have a function of switching over ultrasound images displayed on the monitor in accordance with the position of a lesion or a region of interest. For example, this switching function allows changing a display range in a depth direction (display depth), rotating and shifting horizontally the ultrasound image on the monitor. In response to a switching operation that an operator has made, the ultrasound observation device performs a corresponding display switching process, and displays the resultant ultrasound image on the monitor.

To facilitate the switching operation, Japanese Patent laid-open Publication No. 2002-315753 discloses an ultrasound diagnostic apparatus having a console with a track ball for rotating or moving the image step by step and continuously. There is also disclosed an ultrasound diagnostic system having a special operating member (a dial, for example) to change the display depth (see, Japanese Patent laid-open Publication No. 2007-313202).

These rotary operating members generate a switching signal (pulse) every time they are rotated by a predetermined angle, and the display depth or the like is determined according to the number of the switching pulses. On the other hand, the display switching process is performed by a single processing circuit. Once the display switching process is started, the single processing circuit cannot accept the subsequent switching pulse until the completion of the running process. Accordingly, the operating member has to be rotated continuously, otherwise only a part of the switching pulses is accepted, and the display on the monitor becomes different from the one intended. In view of this, the conventional ultrasound observation device gives a fixed waiting time to each switching pulse, so that the display switching process is not started until the waiting time elapsed after the last switching pulse is generated. However, the waiting time after each switching pulse prolongs the time taken to switch the display of the ultrasound image, and annoys the operator.

In addition, since every operator makes the switching operation at different speed, the fixed waiting time may be inconvenient to some operators. For example, when a relatively short waiting time is established on the basis of a fast operator, the slow operators have trouble to make the switching operation fast enough to generate the next switching pulse within the waiting time. In this instance, the switching operation has to be made once again after the already-initiated display switching process is completed. By contrast, when a relatively long waiting time is established on the basis of the slow operator, the fast operators have to wait long after the switching operation.

### SUMMARY OF THE INVENTION

In view of the foregoing, it is a main object of the present invention to provide an ultrasound observation device and a method for controlling operation thereof to establish an optimum waiting time according to switching operation speed.

Another object of the present invention is to provide an ultrasound observation device and a method for controlling operation thereof to reduce the time taken to switch the display of an ultrasound image.

In order to achieve the above and other objects, the ultrasound observation device according to the present invention includes a monitor for displaying an ultrasound image, an operating member to be operated for switching the display of the ultrasound image, a pulse generator, a detector for detecting a generation state of the switching pulse, a waiting time determiner, and a processor for executing display switching process. The pulse generator generates a switching pulse every time the operating member is operated by a predetermined amount. Based on the generation state of the switching pulse, the waiting time determiner determines a waiting time T to start the display switching process of the ultrasound image after generation of each switching pulse. When the waiting time elapsed after the generation of the switching pulses stops, the processor executes the display switching process corresponding to the number of the switching pulses generated.

Preferably, the detector includes a timer for measuring generation intervals Δtᵢ (i = 1, 2, ···, n-1) between each of the switching pulses. Based on the generation intervals Δtᵢ, the waiting time determiner determines the waiting time T.

In one aspect of the present invention, the detector further includes a counter for counting the number n of the switching pulses. The waiting time determiner determines the waiting time T, based on the average of the generation intervals Δtᵢ calculated by ΣΔtᵢ / n-1.

Alternatively, the waiting time T may be determined, based on the latest generation interval Δtₙ₋₁ of the switching pulses or the longest generation interval Δtₘₐₓ among the generation intervals Δtᵢ. Still alternatively, the waiting time T may be determined by adding an additional time Tα to on one of the average of the generation intervals Δtᵢ, the latest generation interval Δtₙ₋₁, and the longest generation interval Δtₘₐₓ.

Preferably, the processor terminates the ongoing display switching process when a switching pulse is generated during the display switching process, and starts new display switching process under the conditions corresponding to the total number of the switching pulses generated.

It is also preferred that the detector detects whether or not the operating member is being operated, so as to detect stoppage of generation of the switching pulses. For this purpose, the pulse generator preferably includes first and second rotary discs rotated by the operating member, switching pulse cutouts arranged at regular intervals on the first rotary disc, rotation detection cutouts arranged on the second rotary disc at regular intervals shorter than the intervals of the switching pulse cutouts, a first photoelectronic sensor for detecting the switching pulse cutouts and generating the switching pulses, and a second photoelectronic sensor for detecting the rotation detection cutouts and generating rotation detection pulses. The detector uses the rotation detection pulses to detect whether or not the operating member is being operated.

The display switching process is preferably one of display depth change, rotation and horizontal shift of the ultrasound image.

In another aspect of the present invention, an ultrasound observation device includes the monitor, the operating member, the pulse generator, the detector, a memory, the waiting time determiner, and the processor for executing display switching process. The memory stores the generation state of the switching pulse as an operation history of the operating member. The waiting time determiner determines a waiting time T, based on the operation history in the memory.

Preferably, the memory stores the operation history for each operator. Still preferably, the memory stores generation intervals Δtᵢ (i = 1, 2, ···, n-1) between each of n-switching pulses as the operation history. In this case, the waiting time determiner determines the waiting time T based on the average of said generation intervals Δtᵢ calculated by ΣΔtᵢ / n-1.

In still another aspect of the present invention, an ultrasound observation device includes the monitor, the operating member, the pulse generator, and the processor that starts display switching process immediately after the generation of a switching pulse. When a subsequent switching pulse is generated during the display switching process, the processor terminates the ongoing display switching process and starts new display switching process under the conditions corresponding to the total number of the switching pulses generated.

In yet another aspect of the present invention, an ultrasound observation device includes the monitor, the operating member, the pulse generator, a first for detecting the generation state of the switching pulse, a second detector for detecting whether or not the operating member is being operated, and the processor. The processor starts the display switching process when the first detector detects the switching pulse and when the second detector detects that the operating member is not operated. On the other hand, the processor does not start the display switching process when the first detector detects the switching pulse and when the second detector detects that the operating member is operated.

A method for controlling operation of an ultrasound observation device, according to the present invention includes the steps of generating a switching pulse when an operating member is operated, detecting a generation state of the switching pulse, determining a waiting time T, and executing the display switching process. The operating member is operated to switch the display of the ultrasound image. The switching pulse is generated every time the operating member is operated by a predetermined amount. In the waiting time determining step, the waiting time T to start the display switching process after generation of each said switching pulse is determined based on the generation state of the switching pulse. The display switching process corresponding to the number of the switching pulses generated is executed when the waiting time elapsed after the generation of the switching pulses stops.

In another aspect of the present invention, a method for controlling operation of an ultrasound observation device includes the steps of generating a switching pulse, detecting a generation state of the switching pulse, storing the generation state of the switching pulse as an operation history of an operating member, determining a waiting time T, and executing display switching process. In the waiting time determining process, the waiting time T to start the display switching process after generation of each switching pulse is determined based on the operation history.

According to the present invention, the waiting time T to start the display switching process is determined according to a switching pulse generation state. Additionally, an optimum waiting time T is established according to the operation speed of the operating member. Further, the display switching process is started in shorter time than the conventional case using a fixed waiting time. It is therefore possible to reduce the time taken to switch the display of an ultrasound image, and to avoid annoying the operator.

The switching pulse generation state is recorded as an operation history, and the waiting time T is determined on the basis of the operation history. This allows establishing an optimum waiting time T that reflects the past switching pulse generation states. Further, the waiting time T is determined in accordance with the generation interval of the switching pulses. It is therefore possible to establish an optimum waiting time T separately for each of the operators who make the switching operation at different speed.

Since the display switching process is started immediately after the generation of the switching pulse, the time taken to switch the display of the ultrasound image can be much reduced.

The second detector detects whether or not the operation member is being operated, and serves to prevent the display switching process from starting in the middle of the switching operation. It is possible to prevent repetition of the switching operation, and reduce the time taken for display switching and annoyance for the operator.

### BRIEF DESCRIPTION OF THE DRAWINGS

The above objects and advantages of the present invention will become more apparent from the following detailed description when read in connection with the accompanying drawings, in which:
FIG. 1 is a block diagram of an ultrasound diagnostic apparatus according to the present invention;
FIG. 2 is a plan view of a console with a depth knob;
FIG. 3A is an explanatory view of an ultrasound image at a shallow display depth;
FIG. 3B is an explanatory view of an ultrasound image at a deep display depth;
FIG. 4 is an explanatory view of waiting time and switching pulses;
FIG. 5 is a flow chart for changing the waiting time;
FIG. 6 is a block diagram of an ultrasound diagnostic apparatus according to the second embodiment of the present invention;
FIG. 7 is an explanatory view of waiting time and switching pulses in the second embodiment;
FIG. 8 is a flow chart for changing the waiting time and updating operation history data;
FIG. 9 is a flow chart of a display switching process without the waiting time;
FIG. 10 is a flow chart in the second embodiment for processing the switching pulse generated after the waiting time;
FIG. 11 is a schematic perspective view of a depth knob and rotary encoders;
FIG. 12 is a plan view of a console with depth buttons; and
FIG. 13 shows a touch screen monitor and a depth window displayed thereon.

### DETAILED DESCRIPTION OF THE PREFERRED EMBODIMENTS

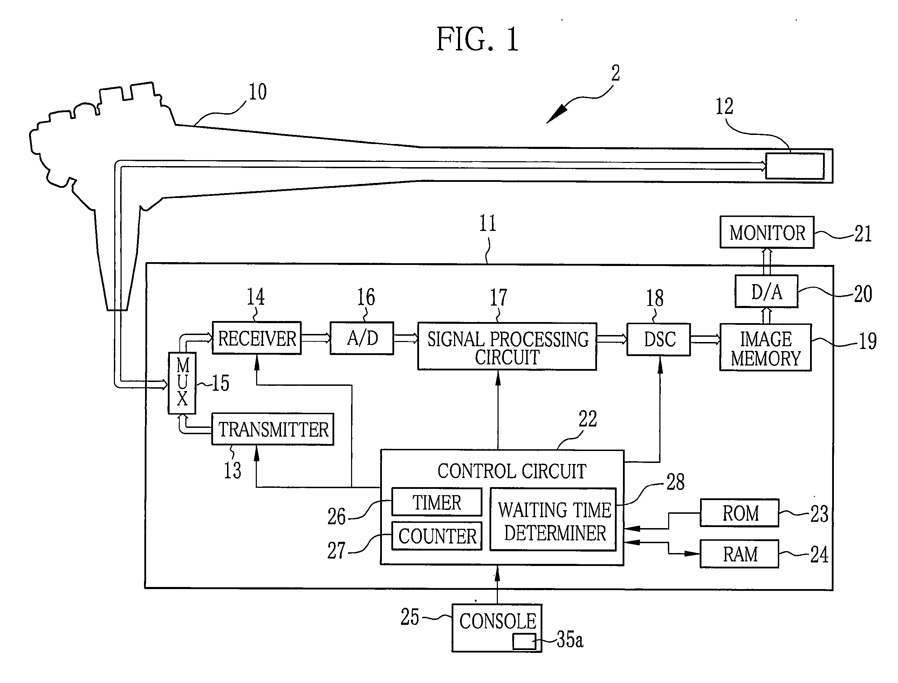

Referring to FIG. 1, an ultrasound diagnostic apparatus 2 includes an ultrasound endoscope (hereinafter, endoscope) 10 and an ultrasound observation device 11 (hereinafter, observation device) connected to the endoscope 10. The ultrasound diagnostic apparatus 2 radiates ultrasound waves into a target region and receives the reflections as echo signals. Then, the ultrasound diagnostic apparatus 2 applies various types of signal processing to the echo signals, and generates an ultrasound image that is a representation of an internal structure of the target region. This ultrasound image is displayed on a monitor 21 connected to the observation device 11.

The endoscope 10 has a long slender insertion section to be inserted into a patient's body cavity, and a CCD imager (not shown) for capturing an optical image and an ultrasound transducer array 12 for obtaining an ultrasound image both provided at a distal end of the insertion section. An optical image captured by the CCD imager is transmitted to an endoscope processor (not shown) connected to the endoscope 10, and applied to the signal processing, and then displayed as an endoscopic image on a monitor device (not shown).

The ultrasound transducer array 12 is composed of a plurality of transducer elements arranged at regular intervals on a backing material having, for example, a convex shape. As is well known, each transducer element oscillates in response to an excitation signal transmitted from a transmitter 13 of the observation device 11, and radiates ultrasound waves into an opposing target region. Each transducer element also receives an echo signal, which is the ultrasound waves reflected off the target region, and transmits the echo signal to a receiver 14 of the observation device 11. The transmitter 13 and the receiver 14 are coupled alternately to the transducer elements through a multiplexer (MUX) 15. The MUX 15 selects and activates adjacent N-transducer elements (several to dozens) at once. At every single sequence of transmission and reception of the ultrasound wave and the echo signal, the MUX 15 shifts one to several transducer elements to be activated in a certain direction. In the case of shifting two transducer elements at a time, for example, each transducer element is activated N/2 times.

N-numbers of the echo signals received at once on the receiver 14 are digitized in an A/D converter 16, and transmitted to a signal processing circuit 17. The signal processing circuit 17 delays the echo signals of each transducer element (in this instance, N/2 echo signals) by an appropriate period so as to bring all the echo signals in phase, and sums up the echo signals. Then, the signal processing circuit 17 applies signal processing, such as a filter process to remove ultrasound carrier components and a log compression process to adjust gain and a dynamic range, to the summed echo signal. The signal processing circuit 17 applies final image processing, including sensitivity time control (STC) in accordance with a propagation distance (depth) of the ultrasound waves, and generates ultrasound image data.

The ultrasound image data is transmitted to a digital scan converter (DSC) 18. The DSC 18 performs raster conversion to convert the ultrasound image data into NTSC data, and stores the data in an image memory 19. This ultrasound image data is retrieved by a D/A converter 20 and converted again into an analog signal, and displayed as an ultrasound image on the monitor 21.

The observation device 11 is controlled entirely by a control circuit 22. The control circuit 22 sends a reference pulse to the transmitter 13 and the receiver 14, and controls the transmission and reception timing of the excitation signal and the echo signal. In response to an operation to switch the display of an ultrasound image, the control circuit 22 generates the corresponding ultrasound image data by adjusting the voltage of the excitation signal to change the propagation distance of the ultrasound waves (ultrasound power), and controlling the signal process in the signal processing circuit 17 and the conversion process in the DSC 18.

The control circuit 22 is connected to a ROM 23, a RAM 24 and a console 25. The ROM 23 is a flash memory, for example, and stores various operating programs and data for operating the ultrasound diagnostic apparatus 2. The control circuit 22 retrieves necessary programs and data from the ROM 23 to the RAM 24, which is a working memory, and runs these programs to control each component of the observation device 11. The ROM 23 also stores a plurality of parameters for operating conditions (excitation signal voltage levels, transmission and reception timings, etc) for the transmitter 13 and the receiver 14, signal processing conditions for the signal processing circuit 17 and conversion process conditions for the DSC 18.

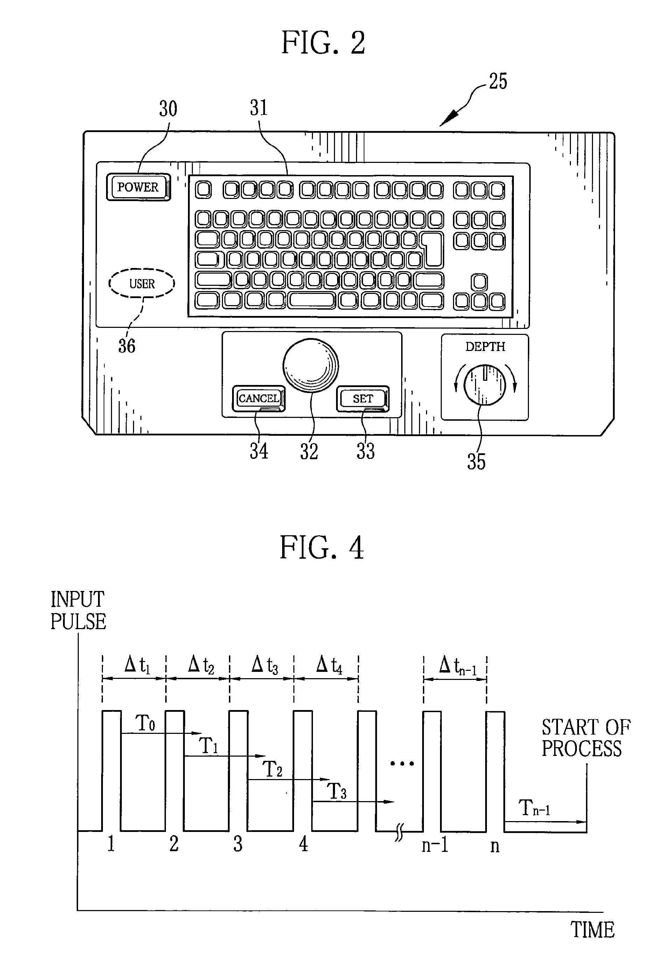

The console 25 is a user interface of the observation device 11, and enters various command signals into the control circuit 22. As shown in FIG. 2, the console 25 includes a power switch 30 for turning on/off the ultrasound diagnostic apparatus 2, an alphabetic key set 31 for entering setup information and the like, a track ball 32 for moving a cursor or a pointer on the monitor 21, a set button 33 for confirming an operation entry, and a cancel button 34 for cancelling the entry.

The console 25 is also equipped with a depth knob 35 for switching (changing) a display range in a depth direction (hereinafter, display depth) of the ultrasound image on the monitor screen. Clockwise rotation of the depth knob 35 increases the display depth, and counterclockwise rotation decreases the display depth. The depth knob 35 is linked to a pulse generator 35a. The depth knob 35 clicks every predetermined rotation angle, and the pulse generator 35a generates a switching pulse (switching signal) each time the depth knob 35 clicks. A positive voltage switching pulse (positive pulse) is generated upon clockwise rotation of the depth knob 35, and a negative voltage switching pulse (negative pulse) is generated upon counterclockwise rotation of the depth knob 35.

The control circuit 22 determines the display depth based on a generation state (positive or negative, and the number) of the switching pulses. The control circuit 22 retrieves the conditions corresponding to the determined display depth from the ROM 23, and activates the transmitter 13, the receiver 14, the signal processing circuit 17 and the DSC 18 under these conditions. The ultrasound image is displayed at the determined display depth on the monitor 21.

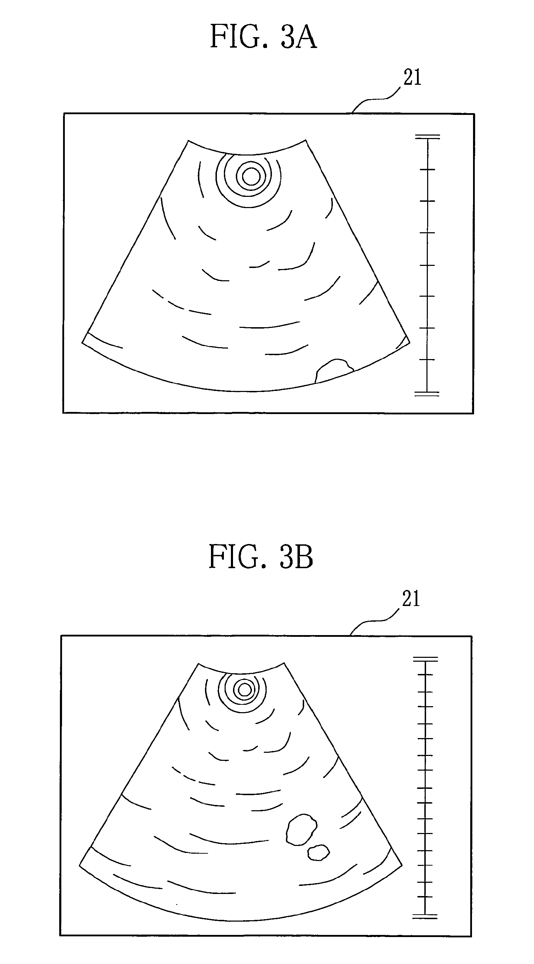

For example, when an ultrasound image is displayed on the monitor 21 as shown in FIG. 3A and the depth knob 35 is rotated clockwise, the control circuit 22 increases the ultrasound power by the level corresponding to the amount of rotation, and transmits the ultrasound waves to an even deeper area of the target region. The control circuit 22 conducts the signal processing and the conversion process according to the display depth, and generates ultrasound image data to display an ultrasound image on the monitor 21. The display on the monitor 21 is now switched to the one shown in FIG. 3B at a deeper display depth.

In contrast, when an ultrasound image is displayed on the monitor 21 as shown in FIG. 3B and the depth knob 35 is rotated counterclockwise, the control circuit 22 decreases the ultrasound power, and changes the conditions of the signal processing and the conversion process. The display on the monitor 21 is now switched to the one shown in FIG. 3A at a shallower display depth. Along with this display depth change, a scale on the right side of the monitor screen is also changed to show current magnification of the ultrasound image. Meanwhile, the depth knob 35 may be configured to decrease the display depth upon clockwise rotation and increase the display depth upon counterclockwise rotation.

Referring back to FIG. 1, the control circuit 22 includes a timer 26, a counter 27 and a waiting time determiner 28. The counter 27 counts the number of the switching pulses. The waiting time determiner 28 determines a waiting time to start the signal processing every time the switching pulse is generated. Within the waiting time, the subsequent switching pulse is acceptable. The timer 26 counts a lapse of the waiting time.

When a first switching pulse is generated, the waiting time determiner 28 sets a preset initial value of the ROM 23 as a waiting time T₀. On the basis of an average operating speed of the depth knob 35, this initial value is determined to 100 msec, for example. When the timer 26 counts up the waiting time T₀ absent a second switching pulse, the control circuit 22 retrieves the conditions corresponding to one switching pulse from the ROM 23. Under these conditions, the control circuit 22 performs the display switching process, and generates ultrasound image data of a different display depth. An ultrasound image of this ultrasound image data is displayed on the monitor 21, and the display depth is switched.

When a second switching pulse is generated within the waiting time T₀, on the other hand, the control circuit 22 enters a count of the timer 26 at that point into the waiting time determiner 28, and resets and starts the timer 26. Every time a switching pulse is generated within the waiting time T, the control circuit 22 enters a count of the timer 26 into the waiting time determiner 28, and resets and starts the timer 26.

The waiting time determiner 28 determines a waiting time Tᵢ based on the cont entered from the timer 26. For example, when n-switching pulses are generated and transmitted sequentially to the control circuit 22, the waiting time determiner 28 stores a generation interval Δtᵢ of each two successive switching pulses through the first to n-th switching pulses (i = 1, 2, ···n-1). These generation intervals Δtᵢ are averaged (ΣΔtᵢ / n-1), and a predetermined additional time T_{α} is added to this average value. The waiting time determiner 28 sets the calculated value as the waiting time T {(ΣΔtᵢ / n-1) + T_{α}}. The additional time T_{α} gives a margin of time to allow the generation interval to slightly exceed the average value, and is assigned an appropriate value.

Practically, the waiting time T is determined every time the switching pulse is generated. As shown in FIG. 4, when the second switching pulse is generated within the waiting time To, a waiting time T₁ is determined by adding the additional time T_{α} to the generation interval Δt₁ between the first and second switching pulses. This generation interval Δt₁ is temporarily stored in a memory such as the RAM 24. When a third switching pulse is generated within the waiting time T₁ that has started upon the generation of the second switching pulse, the waiting time determiner 28 firstly retrieves the generation interval Δt₁ from the RAM 24, and calculates the average generation interval by adding a generation interval Δt₂ between the second and third switching pulses to the generation interval Δt₁ and dividing the sum by the number of pulse intervals (3-1=2). Then, the waiting time determiner 28 adds the additional time T_{α} to this average generation interval to determine a waiting time T₂. The generation interval Δt₂ is then temporarily stored in the RAM 24.

When a forth switching pulse is generated within the waiting time T₂ after the third switching pulse, the waiting time determiner 28 retrieves the generation intervals Δt₁ and Δt₂ from the RAM 24, and calculates the average generation interval by adding an generation interval Δt₃ between the third and forth switching pulses to the generation intervals Δt₁, Δt₂ and dividing the sum by the number of pulse intervals (4 - 1 = 3). Then, the additional time T_{α} is added to the average generation interval, and the sum is determined as a waiting time T₃. When a waiting time Tₙ₋₁ elapsed after the generation of the n-th switching pulse, the display switching process is started according to n-switching pulses. At the same time, the counter 27 and the previous generation intervals in the RAM 24 are reset. For decimal numbers in the average generation interval, it is preferred to decide how to process the decimal numbers (rounding up, rounding down or such) previously.

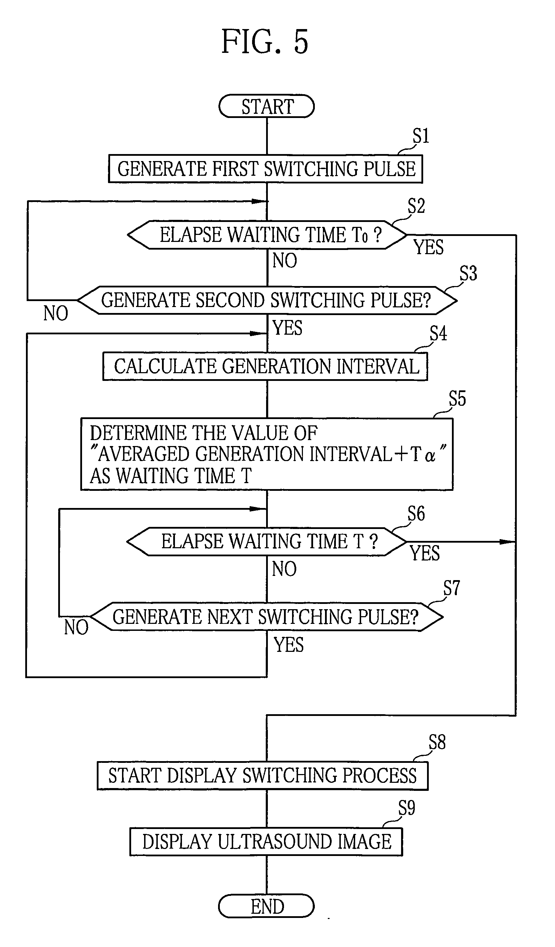

Next, with reference to a flow chart of FIG. 5, the operation of the above observation device 11 is explained. When the depth knob 35 is rotated by one click, the pulse generator 35a working together with the depth knob 35 generates and transmits a first switching pulse to the control circuit 22 (S1). For the first switching pulse, the initial value (100 msec) in the ROM 23 is determined as the waiting time To, and the timer 26 is started. When the waiting time T₀ elapsed without the generation of a second switching pulse (YES in S2), the display switching process is started under the conditions corresponding to one switching pulse (S8).

When the depth knob 35 is rotated by one more click, the pulse generator 35a generates the second switching pulse. If the second switching pulse is generated within the waiting time T₀ (YES in S3), the waiting time determiner 28 calculates the generation interval Δt₁ (S4), and determines the waiting time T₁ using the generation interval Δt₁ (S5). This sequence of steps S4 and S5 is repeated every time a subsequent switching pulse is generated within the current waiting time T, and a new waiting time T is determined (NO in S6).

No switching pulse is generated without further rotation of the depth knob 35. When the current waiting time T elapsed absent of a subsequent switching pulse (YES in S6), the display switching process corresponding to the sum total of the switching pulses generated is started (S8), and the display of the ultrasound image is switched on the monitor 21 (S9).

The depth knob 35 returns to the initial position as the operator releases his hand from it. When the depth knob 35 is rotated again by one click, the pulse generator 35a generates a first switching pulse to start the display switching process corresponding to one switching pulse. Alternatively, the depth knob 35 may be configured to stay in the rotated position. In this case, the counter 27 is not reset. When the depth knob 35 is rotated again by several clicks, the pulse generator 35a generates the same number of switching pulses as the clicks. These new switching pulses are added to the previous switching pulses, and the display switching process is executed according to the total number of the switching pulses.

Instead of using the average generation interval, the latest generation interval (between the last and previous switching pulses) may be used for determining the waiting time T. In this case, every time the switching pulse is generated, the waiting time T is determined by adding a count of the timer 26 (namely, a generation interval Δtₙ₋₁ between the this time' s switching pulse and the previous switching pulse) and the additional Time T_{α}.

Alternatively, the longest generation interval may be used for determining the waiting time T. In this case, the generation interval Δt₁ between the first and second switching pulses is stored in the RAM 24, as in the case of the average generation interval. The generation interval in the RAM 24 is defined as a generation interval Δtₘₐₓ, which is only overwritten by a longer generation interval. The waiting time T is determined by adding the generation interval Δtₘₐₓ and the additional Time T_{α}. This configuration can eliminate the process to calculate the average generation interval. Furthermore, it may be possible to allow the operators to select one of the average generation interval (ΣΔtᵢ / n-1), the latest generation interval (Δtₙ₋₁) and the longest generation interval (Δtₘₐₓ).

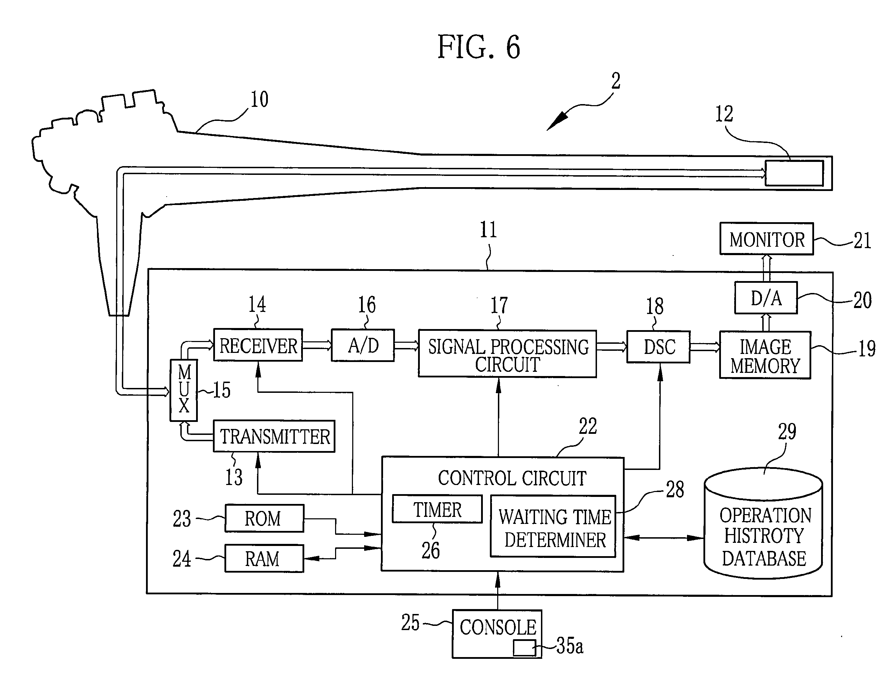

Although the waiting time is calculated for each switching operation in the above embodiment, it may be calculated for a series of switching operations. In this instance, as shown in FIG. 6, an operation history database 29 is used to store a plurality of past generation intervals as an operation history. In FIG. 6, the elements similar to those in the first embodiment are designated by the same reference numerals, and the detailed explanations thereof are omitted.

In FIG. 6, the control circuit 22 includes the timer 26, the waiting time determiner 28 and an operation history database 29. The operation history database 29 stores the previous n-generation intervals, measured on the timer 26, as operation history data. Every time the switching operation is performed to create new data, the oldest operation history data is deleted. The number of the operation history data, or the generation intervals, being stored is predetermined appropriately in accordance with the capacity of the operation history database 29.

The control circuit 22 operates the waiting time determiner 28 to determine the waiting time T upon or before the generation of a first switching pulse. The waiting time determiner 28 retrieves the generation intervals Δtᵢ (i=1, 2, ···n-1) from the operation history database 29. The waiting time determiner 28 calculates the average of the generation intervals Δtᵢ (ΣΔtᵢ /n-1), and adds an additional time T_{α} retrieved from the ROM 23 to the average generation interval to determine the waiting time T {(ΣΔtᵢ / n-1) + T_{α}}.

For example, providing that the generation intervals of ten switching pulses be stored as the operation history data, nine generation intervals Δt₁ to Δt₉ are stored in the operation history database 29. The waiting time determiner 28 retrieves these nine generation intervals Δt₁ to Δt₉ from the operation history database 29, and determines the calculation of (Δt₁ + Δt₂ + ··· Δt₉) / 9 + T_{α} as the waiting time T. The ROM 23 stores an initial value of the waiting time T. When there is no operation history data stored in the operation history database 29, this initial value is set as the waiting time T.

When the waiting time T elapsed without a second switching pulse, the control circuit 22 retrieves the conditions corresponding to one switching pulse from the ROM 23. Then, the control circuit 22 performs the display switching process under the retrieved conditions, and generates ultrasound image data of different display depth. An image of this ultrasound image data is displayed on the monitor 21, and the display depth is switched.

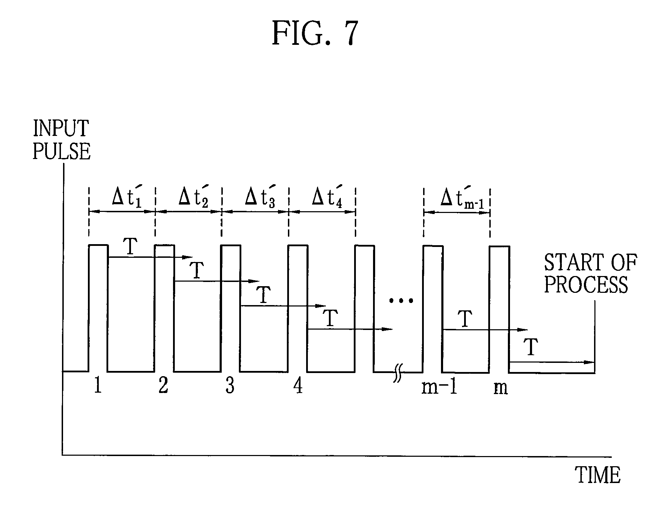

On the other hand, as shown in FIG. 7, when the second switching pulse is generated within the waiting time T, the control circuit 22 temporarily stores a count of the timer 26 at that point, or namely a generation interval Δt'₁, between the first and second switching pulses, in the RAM 24. Then, the control circuit 22 resets and starts the timer 26. When a third switching pulse is generated within the waiting time T that has started upon the generation of the second switching pulse, the control circuit 22 stores a generation interval Δt'₂ between the second and third switching pulses in the RAM 24, and resets and starts the timer 26. In this manner, every time a subsequent switching pulse is generated at a shorter interval than the waiting time T, a count at that point is stored and the timer 26 is re-started. In the end, the RAM 24 stores a plurality of generation intervals Δt'ⱼ (j = 1, 2, ··· m-1) between m-switching pulses (m ≤ n) generated through a series of switching operations.

When the waiting time T elapsed after the generation of the m-th switching pulse, the control circuit 22 retrieves the conditions corresponding to the sum total of the switching pulses generated, and starts the display switching process under these conditions. At the same time, the control circuit 22 replaces the old operation history data with the generation intervals Δt'ⱼ temporarily stored in the RAM 24. If the number (m) of the switching pulses is less than the set number (n) of the switching pulses to be stored in the operation history database 29 (m < n), all the generation intervals Δt'ⱼ are stored. The old operation history data of the same number is then deleted from the operation history database 29. By contrast, if the number (m) of the switching pulses is the same as the preset number (n) of the switching pulses (m = n), all the generation intervals Δt'ⱼ are stored as the latest generation intervals Δtᵢ, and all the old operation history data is deleted from the operation history database 29.

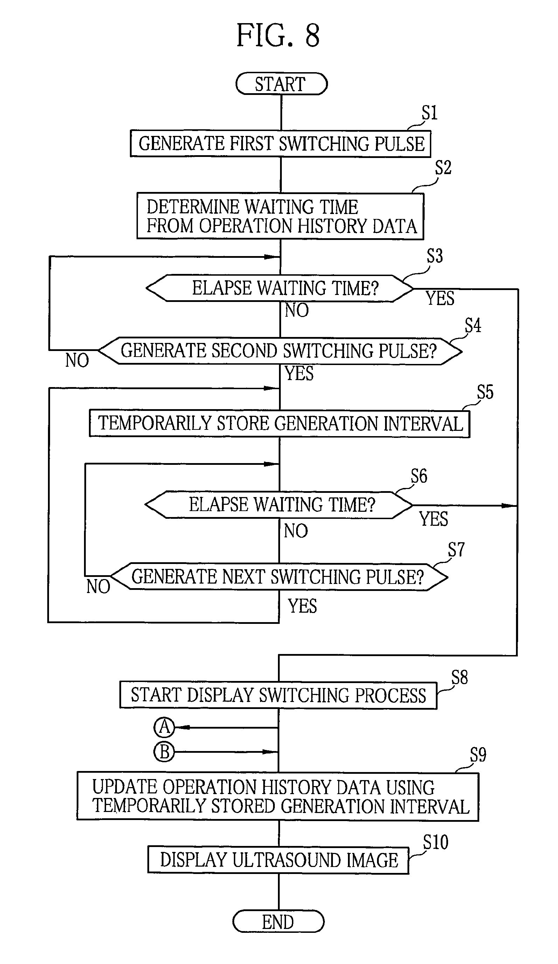

Next, with reference to a flow chart of FIG. 8, the operation of this second embodiment device is explained. When a first switching pulse is generated from the pulse generator 35a upon rotation of the depth knob 35 (S1), the waiting time determiner 28 retrieves the operation history data from the operation history database 29, and determines the waiting time T (S2). The control circuit 22 starts the timer 26. When the waiting time T elapsed without the generation of a second switching pulse (YES in S3), the display switching process is started under the conditions corresponding to one switching pulse (S8). On the other hand, when the second switching pulse is generated within the waiting time T (YES in S4), a count of the timer 26 at that point is temporarily stored as a generation interval Δt'₁ in the RAM 24 (S5) . The control circuit 22 resets and starts the timer 26.

After the generation of the second switching pulse, when the waiting time T elapsed without a third switching pulse (YES in S6), the display switching process is started under the conditions corresponding to two switching pulses (S8). By contrast, when the third switching pulse is generated within the waiting time T (YES in S7), a count of the timer 26 at that point is stored as a generation interval Δt'₂ in the RAM 24 (S5), and the timer 26 is re-started.

Thereafter, when the waiting time T elapsed without the generation of a subsequent switching pulse (YES in S6), the display switching process is started under the conditions corresponding to the sum total of the switching pulses generated (S10), and the display of the ultrasound image is switched on the monitor 21. Along with the display switching process, the control circuit 22 updates the operation history data in the operation history database 29 using the generation intervals Δt'ⱼ temporarily stored in the RAM 24 (S9). The updated operation history data is retrieved in the next switching operation to determine the waiting time T. The temporarily stored generation intervals Δt'ⱼ are deleted from the RAM 24 at the end of the step S10, or any other appropriate time.

As in the first embodiment, the latest generation interval Δtₙ₋₁ (between the last and previous switching pulses) may be used to determine the waiting time T. In this case, every time the switching pulse is generated, the count temporarily stored in the RAM 24 is overwritten with a count of the timer 26 at that time. In the end, only the last-stored generation interval Δtₙ₋₁ is stored in the operation history database 29. This configuration allows the operation history database 29 to have small capacity. Also, the process to calculate the average generation interval can be eliminated. Enabling to use the RAM 24 as the operation history database and eliminating the operation history database 29 itself, this configuration provides a cost advantage.

Alternatively, the longest generation interval Δtₘₐₓ may be used for determining the waiting time T. This configuration also enables reducing the capacity of the operation history database 29. In this case, as described above, every time the switching pulse is generated, a count of the timer 26 at that point is compared with a count temporarily stored in the RAM 24, and the count in the RAM 24 is overwritten only when the count of the timer 26 is greater. In the end, only the longest generation interval Δtₘₐₓ is stored in the operation history database 29. It is further possible to allow the operators to select one of the average generation interval (ΣΔtᵢ / n-1), the latest generation interval (Δtₙ₋₁) and the longest generation interval (Δtₘₐₓ).

Operator names may be recorded beforehand in the operation history database 29, and the operation history data may be organized under operator names. In this case, the console 25 is equipped with a "user" button 36, shown by a dashed-line in FIG. 2, to select an operator. In the operation history database 29, the operation history data is associated with the operator names. In using the ultrasound diagnostic apparatus 2, an operator first selects his/her own name using the "user" button 36. The waiting time determiner 28 retrieves the operation history data under the selected operator name from the operation history database 29, and determines the waiting time T. At the end of the display switching operation, the generation intervals Δt'ⱼ temporarily stored in the RAM 24 are stored in connection with the selected operator name in the operation history database 29. With this configuration, the waiting time is determined appropriately for each of the operators.

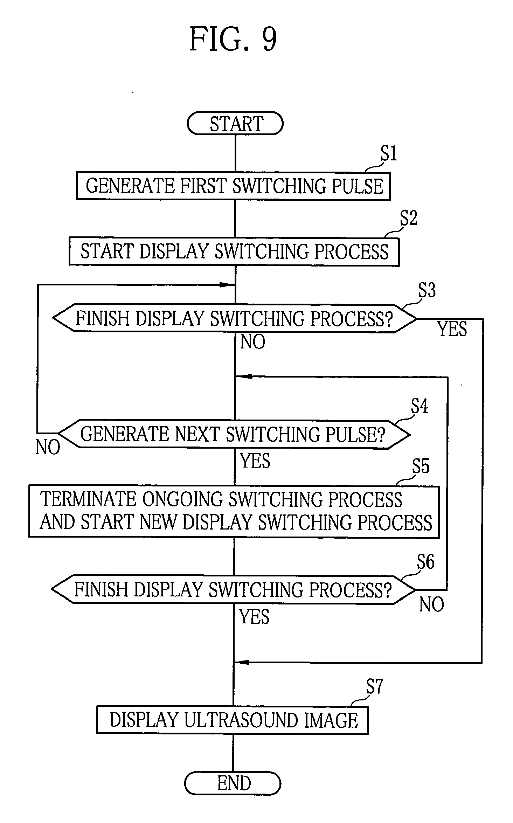

Although the above embodiments are described based on the presence of the waiting time, eliminating the waiting time also reduces the time taken to switch the display of the ultrasound image. FIG. 9 shows a flow chart of signal processing without the waiting time. When a first switching pulse is generated (S1), the control circuit 22 starts the display switching process immediately (S2). When a second switching pulse is generated during the display switching process (YES in S4), the control circuit 22 terminates the ongoing display switching process, and begins the display switching process again under the condition corresponding to two switching pulses (S5). Every time the switching pulse is generated during the display switching process, the control circuit 22 repeats the step S5, and displays a new ultrasound image on the monitor 21 as soon as the display switching process is completed (S7). This configuration eliminates the timer 26, the counter 27, the waiting time determiner 28 and the operation history database 29, and is still able to reduce the time taken to switch the display.

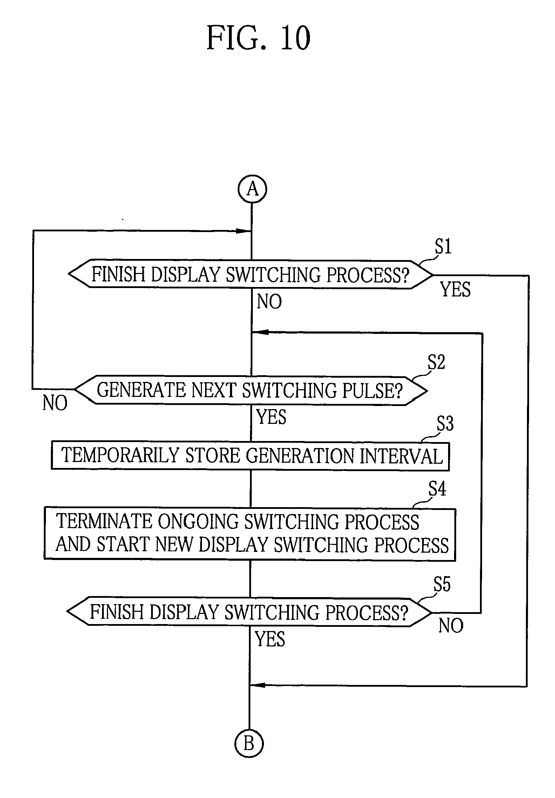

This third embodiment can be incorporated in the first and second embodiments. For the first embodiment, the ongoing display switching process is terminated when a switching pulse is generated during the display switching process started after the waiting time T (S8 in FIG. 5), and the display switching process is begun again under the conditions corresponding to the sum total of the switching pulses generated. For the second embodiment, as shown in FIG. 10, the control circuit 22 temporarily stores a count of the timer 26 at that point in the RAM 24 (S3), and terminates the ongoing process when a switching pulse is generated during the display switching process (NO in S1), and begins the display switching process again under the conditions corresponding to the sum total of the switching pulses generated (S4). In both embodiments, a switching pulse can be accepted even in the middle of the display switching process.

The time taken to switch the display can also be reduced by detecting the rotation of the depth knob mechanically. In this case, the pulse generator is composed of, for example, two sets of rotary encoders each having a rotary disk and a photoelectronic sensor.

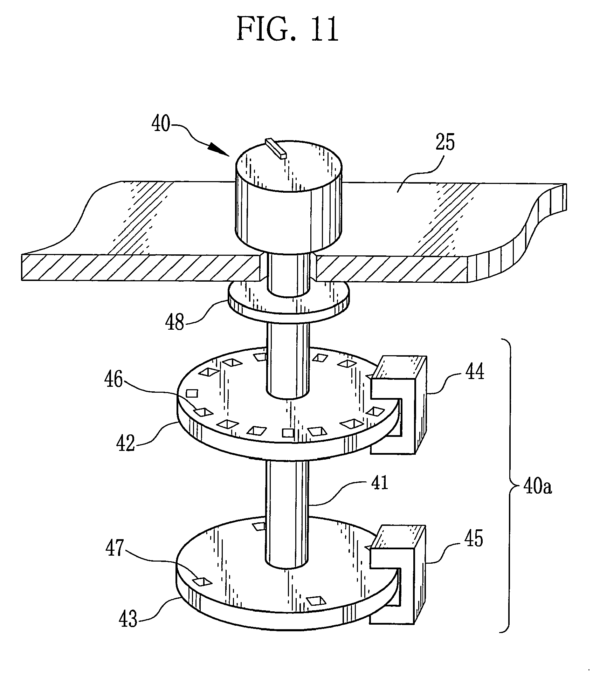

As shown in FIG. 11, a depth knob 40 includes a shaft 41 that projects into the console 25. In the middle and at a distal end of this shaft 41, there are attached two rotary disks 42, 43, one for rotation detection and the other for switching pulse generation. These rotary disks 42, 43 have regularly spaced cutouts 46, 47 respectively. The cutouts 46 in the rotary disk 42 are spaced at shorter intervals than the cutouts 47 in the rotary disk 43 (for example, 1/5 pitch of the cutouts 47). Two photoelectronic sensors 44, 45, one for rotation detection and the other for switching pulse generation are arranged to accept the rotary disks 42, 43. Two rotary encoders composed of the rotary disks 42, 43 and the photoelectronic sensors 44, 45 constitute a pulse generator 40a. The photoelectronic sensor 44 is a common transmissive sensor having a U-shaped cross section, and its light emitting element and light receiving element are arranged above and below to face each other across the edge of the rotary disk 42. The photoelectronic sensor 45 has the same structure as the photoelectronic sensor 44. A reference numeral 48 in the drawing denotes a retaining flange. For the sake of simplicity, only sixteen cutouts 46 and four cutouts 47 are shown in the drawing, but in fact more cutouts are formed in the rotary disks 42, 43.

When the depth knob 40 is rotated by one click, one cut-out 47 in the rotary disk 43 passes through the photoelectronic sensor 45. The passage of the cut-out 47 raises the output level of the photoelectronic sensor 45 to a "High" level, and thus a switching pulse is generated. During this one click rotation, four cutouts 46 in the rotary disk 42 pass through the photoelectronic sensor 44. The output level of the photoelectronic sensor 45 rises to a "High" level four times accordingly. As long as the output level of the photoelectronic sensor 45 rises and falls, the control circuit 22 judges that the depth knob 40 is being rotated. In this instance, the control circuit 22 does not start the display switching process even if a switching pulse has already been generated, so as to accept a subsequent switching pulse. When the output level of the photoelectronic sensor 44 stabilizes, the control circuit 22 starts the display switching process under the conditions corresponding to the sum total of the switching pulses. This configuration can eliminate the initial value of the waiting time and the waiting time determining process, and is still able to reduce the time taken to switch the display of the ultrasound image, according to the operation on the depth knob 40. Meanwhile, instead of using the rotary encoders, it is possible to install a contact sensor in the depth knob, and detect the completion of the switching operation as the operator releases his hand from the depth knob.

In combining the depth knob 40 with the first embodiment device, store of the input intervals Δtᵢ and the number of the switching pulses should only be performed while the output level of the photoelectronic sensor 44 is rising and falling even if a switching pulse is generated. Then, as the output level of the photoelectronic sensor 44 stabilizes, the average of the input intervals Δtᵢ is calculated, and the waiting time T is determined. It is thereby possible to reduce the number of the waiting time determining process, and the workload on the control circuit 22.

In combining the depth knob 40 with the second embodiment, the display switching process should not be started while the output level of the photoelectronic sensor 44 is rising and falling even if a switching pulse is generated. On occasion, the operator seeks an appropriate display depth while operating the depth knob. Therefore, not to start the display switching process during the operation of the depth knob can add margin to the waiting time T, and give the operator more time to determine an appropriate display depth.

The present invention is suitable for rotary operating members. The present invention is not only applicable to the combination of the depth knob and the pulse generator, but also to a rotation switch for rotating an ultrasound image on the monitor 21 about the center of the ultrasound scan, and a move switch for shifting an ultrasound image horizontally on the monitor 21. In these instances, the corresponding display switching process (rotation or horizontal shift) is applied to ultrasound image data that the DSC 18 generates.

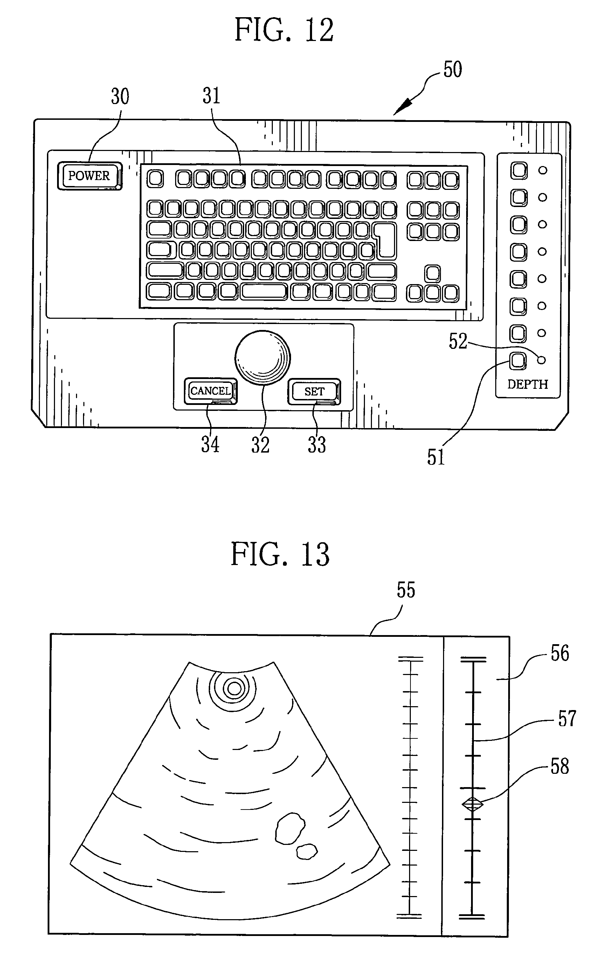

Instead of the depth knobs 35, 40, a depth button may be used as the operating member. As shown in FIG. 12, a console 50 is equipped with a set of depth buttons 51 aligned vertically on the right side of the console 50. Each depth button 51 corresponds to a predetermined display depth, and is integrated with a switch (not shown). These depth buttons 51 enters switching signals of corresponding display depths into the control circuit 22. On the right side of the depth buttons 51, LED lamps 52 are placed. Each LED lamp 52 irradiates upon the press of the corresponding depth button 51, and indicates the current display depth. When a depth button 51 is pressed during the display switching process, the ongoing display switching process is terminated, and new display switching process is started under the conditions corresponding to the depth button 51 being pressed. This configuration allows for directly selecting a desired display depth, and improves operation performance. Additionally, the current display depth is easily identified. The depth button 51 can specify a certain display depth directly, unlike the depth knob, and eliminate the need of the waiting time.

Although the above embodiments use the console having one or more operating members for display depth, it is possible, as shown in FIG. 13, to use a touch panel monitor 55 that displays a depth window 56. This depth window 56 includes a scale 57 of the display depth and a pointer 58 for indicating the current display depth. Moving up or down the pointer 58 with a finger or a touch pen leads to enter a switching signal into the control circuit 22, and the ultrasound image is displayed on the monitor 55 at the display depth corresponding to the position of the pointer 58.

While the above embodiments are directed to the ultrasound endoscope having the ultrasound transducer array and the imaging unit, the present invention is applicable to ultrasound probes only having the ultrasound transducer array. In addition, the ultrasound probes may either be insertion probes to be inserted into a body cavity through a forceps channel of the endoscope, or contact probes to be pressed against a body surface.

Although the present invention has been fully described by the way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An ultrasound observation device (11) comprising:
a monitor (21) for displaying an ultrasound image;
an operating member (35, 40) to be operated for switching the display of said ultrasound image;
a pulse generator (35a, 40a) for generating a switching pulse every time said operating member (35, 40) is operated by a predetermined amount;
a detector (22) for detecting a generation state of said switching pulse;
a waiting time determiner (28) for determining a waiting time T to start display switching process of said ultrasound image after generation of each said switching pulse, based on said generation state of said switching pulse; and
a processor (22) for executing said display switching process corresponding to the number of said switching pulses generated when said waiting time elapsed after the generation of said switching pulses stops.

2. The ultrasound observation device of claim 1, wherein said detector (22) includes a timer (26) for measuring generation intervals Δtᵢ (i = 1, 2, ···, n-1) between each of said switching pulses, and said waiting time determiner (28) determines said waiting time T based on said generation intervals Δtᵢ.

3. The ultrasound observation device of claim 2, wherein said detector (22) further includes a counter (26) for counting the number n of said switching pulses, and said waiting time determiner (28) determines said waiting time T based on the average of said generation intervals Δtᵢ calculated by ΣΔtᵢ / n-1.

4. The ultrasound observation device of claim 2, wherein said waiting time determiner (28) determines said waiting time T based on the latest generation interval Δtₙ₋₁ of said switching pulses.

5. The ultrasound observation device of claim 2, wherein said waiting time determiner (28) determines said waiting time T based on the longest generation interval Δtₘₐₓ among said generation intervals Δtᵢ.

6. The ultrasound observation device of claim 2, wherein said waiting time determiner (28) determines said waiting time T by adding an additional time Tα to one of the average of said generation intervals Δtᵢ calculated by ΣΔtᵢ / n-1, the latest generation interval Δtₙ₋₁ of said switching pulses, and the longest generation interval Δtₘₐₓ among said generation intervals Δtᵢ.

7. The ultrasound observation device of claim 1, wherein said processor (22) terminates ongoing display switching process when said switching pulse is generated during said display switching process, and starts new display switching process under the conditions corresponding to the total number of the switching pulses generated.

8. The ultrasound observation device of claim 1, wherein said detector (22) detects stoppage of generation of said switching pulses by detecting whether or not said operating member (40) is being operated.

9. The ultrasound observation device of claim 8, wherein said pulse generator (40a) comprises:
first and second rotary discs (42, 43) rotated by said operating member (40);
switching pulse cutouts (47) arranged at regular intervals on said first rotary disc (43);
rotation detection cutouts (46) arranged on said second rotary disc (42) at regular intervals shorter than the intervals of said switching pulse cutouts (47);
a first photoelectronic sensor (45) for detecting said switching pulse cutouts (47) and generating said switching pulses; and
a second photoelectronic sensor (44) for detecting said rotation detection cutouts (46) and generating rotation detection pulses,
wherein said detector (22) detects whether or not said operating member (40) is being operated based on said rotation detection pulses.

10. The ultrasound observation device of claim 1, wherein said display switching process is one of display depth change, rotation and horizontal shift of said ultrasound image.

11. An ultrasound observation device (11) comprising:
a monitor (21) for displaying an ultrasound image;
an operating member (35, 40) to be operated for switching the display of said ultrasound image;
a pulse generator (35a, 40a) for generating a switching pulse every time said operating member (35, 40) is operated by a predetermined amount;
a detector (22) for detecting a generation state of said switching pulse;
a memory (29) for storing said generation state of said switching pulse as an operation history of said operating member (35, 40);
a waiting time determiner (28) for determining a waiting time T to start display switching process of said ultrasound image after generation of each said switching pulse, based on said operation history in said memory (29); and
a processor (22) for executing said display switching process corresponding to the number of said switching pulses generated when said waiting time elapsed after the generation of said switching pulses stops.

12. The ultrasound observation device of claim 11, wherein said memory (29) stores said operation history for each operator.

13. The ultrasound observation device of claim 12, wherein said memory (29) stores generation intervals Δtᵢ (i = 1, 2, ···, n-1) between each of n-switching pulses as said operation history, and said waiting time determiner (28) determines said waiting time T based on the average of said generation intervals Δtᵢ calculated by ΣΔtᵢ / n-1.

14. An ultrasound observation device (11) comprising:
a monitor (21) for displaying an ultrasound image;
an operating member (35, 40) to be operated for switching the display of said ultrasound image;
a pulse generator (35a, 40a) for generating a switching pulse every time said operating member (35, 40) is operated by a predetermined amount; and
a processor (22) for executing display switching process corresponding to the number of said switching pulses generated, said processor (22) starting said display switching process immediately after the generation of said switching pulse, and when a subsequent switching pulse is generated during ongoing display switching process, said processor terminating ongoing display switching process and starting new display switching process under the conditions corresponding to the total number of the switching pulses generated.

15. An ultrasound observation device (11) comprising:
a monitor (21) for displaying an ultrasound image;
an operating member (40) to be operated for switching the display of said ultrasound image;
a pulse generator (40a) for generating a switching pulse every time said operating member (40) is operated by a predetermined amount;
a first detector (22) for detecting a generation state of said switching pulse;
a second detector (22) for detecting whether or not said operating member (40) is being operated; and
a processor (22) for executing display switching process corresponding to the number of said switching pulses generated, said processor (22) starting said display switching process when said first detector detects said switching pulse and when said second detector detects that said operating member (40) is not operated, said processor (22) not starting said display switching process when said first detector detects said switching pulse and when said second detector detects that said operating member (40) is operated.

16. A method for controlling operation of an ultrasound observation device (11) comprising steps of:
operating an operating member (35, 40) for switching the display of an ultrasound image;
generating a switching pulse every time said operating member (35, 40) is operated by a predetermined amount;
detecting a generation state of said switching pulse;
determining a waiting time T to start display switching process of said ultrasound image after generation of each said switching pulse, based on said generation state of said switching pulse; and
executing said display switching process corresponding to the number of said switching pulses generated when said waiting time elapsed after the generation of said switching pulses stops.

17. A method for controlling operation of an ultrasound observation device (11) comprising steps of:
operating an operating member (35, 40) for switching the display of an ultrasound image;
generating a switching pulse every time said operating member (35, 40) is operated by a predetermined amount;
detecting a generation state of said switching pulse;
storing said generation state of said switching pulses as an operation history of said operating member;
determining a waiting time T to start display switching process of said ultrasound image after generation of each said switching pulse, based on said operation history; and
executing said display switching process corresponding to the number of said switching pulses generated when said waiting time elapsed after the generation of said switching pulses stops.
